# EUROPÄISCHE PATENTSCHRIFT

(11) **EP 0 408 489 B1**
(45) Veröffentlichungstag und Bekanntmachung des Hinweises auf die Patenterteilung: **14.09.1994**
(21) Anmeldenummer: 90810413.6
(22) Anmeldetag: 07.06.1990
(51) Int. Cl.: A61B 17/58

(54) **Implantat zur Fixierung zweier Wirbel**
Implant for the fixation of two vertebrae
Implant pour la fixation de deux vertèbres

(30) Priorität: 12.07.1989 CH 2610/89
(43) Veröffentlichungstag der Anmeldung: 16.01.1991
(73) Patentinhaber: SULZER Medizinaltechnik AG, 8404 Winterthur (CH)
(72) Erfinder: Jacob, Hilaire, Dr.-Ing., CH-8409 Winterthur (CH); Suezawa, Yoshinori, Prof., Dr.-med., CH-8122 Binz (CH); Schreiber, Adam, Prof., Dr.-med., CH-8700 Küsnacht (CH); Koch, Rudolf, CH-8267 Berlingen (CH)
(74) Vertreter: Hammer, Bruno, Dr.

(56) Entgegenhaltungen:
- EP-A- 0 240 034
- WO-A-87/01026
- DE-U- 8 816 233
- FR-A- 2 615 095

## Beschreibung

Die Erfindung betrifft ein Implantat zur Fixierung zweier Wirbel in ihrer relativen Lage zueinander, bestehend aus zwei jeweils in die beiden Wirbel eingreifenden Verankerungselementen, die über ein den Abstand der Verankerungselemente überbrückendes Stabilisierungselement verbunden sind, wobei das Stabilisierungselement innerhalb eines vorgegebenen Bereiches in einem beliebigen Winkel zum Verankerungselement fixierbar ist.

Spondylodese-Stabilisatoren zur Fixierung der Lage zweier Wirbel sind in recht unterschiedlichen Bauformen bekannt. Sie sollten gleichzeitig sich teilweise widersprechenden Forderungen des Operateurs genügen, um ein Optimum darzustellen. Eine funktionstüchtige fixierte Endlage vorausgesetzt, sollten diese Implantate nacheinstellbar sein, aus wenigen Standardelementen bestehen, keine umfangreichen Vorarbeiten am Knochen erfordern und im Verhältnis zu den Wirbeln kleine und kompakte Dimensionen aufweisen. Die DE-OS 28 34 891 A1 zeigt einen Bausatz, der viel Flexibilität, aber auch viele, raumbeanspruchende mechanische Elemente aufweist. Wesentlich kompakter wirkt eine Ausführung in der CH-PS 646 857, in der jedoch die Forderung besteht, dass die Knochenschrauben zu einem Stabilisierungselement spätestens mit dem Erreichen der Stabilisierungslage in zueinander parallelen Ebenen liegen müssen.

Im weiteren zeigt die Offenlegungsschrift EP-A-0 240 034 einen ausserhalb vom menschlichen Körper gelegenen Fixateur zur Osteosynthese, bei dem mehrere Halterungen auf einem Trägerkörper längs verschiebbar sind und mit einer hohlen Knebelschraube, in die ein Bohrwerkzeug in einer windschiefen Lage zu einem Knochen eingeführt wird, fixierbar ist, um nach dem Bohren in der gleichen Lage eine Knochenschraube durch die Knebelschraube hindurch zu setzen und durch weiteres Anziehen der Knebelschraube diese oder eine daran anschliessende Hülse soweit radial zu deformieren, dass eine Klemmung zur Knochenschraube entsteht. Ein Nachteil dieser Anordnung ist, dass für die zweite Klemmung die Deformation eines nicht sehr stark verformbaren Zwischengliedes notwendig wird, um die Durchmessertoleranzen der Knochenschrauben auszugleichen. Bei einer äusseren, jederzeit kontrollierbaren und nachstellbaren Anordnung ist dies sicher zulässig. Hingegen würde dieses Prinzip - abgesehen von den grossen Abmessungen und der Körperverträglichkeit der Werkstoffe - als Vollimplantat Risiken mit sich bringen.

Aufgabe der Erfindung ist es, innerhalb eines gegebenen Anwendungsbereichs die Lage der Verankerungselemente, z.B. Knochenschrauben, entsprechend den Verankerungsmöglichkeiten an den Wirbeln auch windschief zueinander festzulegen, während der Verstellung des Stabilisierungselementes eine Verstellung der windschiefen Lage zuzulassen und nach Erreichen der Endlage eine starre Verbindung von einem Verankerungselement zum anderen zu gewährleisten. Die windschiefe Lage der Verankerungselemente soll ohne Lösen der Verankerung verstellbar sein. Diese Aufgabe wird durch die Kennzeichen der unabhängigen Ansprüche 1 und 2 gelöst.

Die Vorteile der Erfindung sind darin zu sehen, dass der Operateur Ort und Lage der Verankerungselemente entsprechend dem vorgefundenen Zustand der Wirbel festlegen kann, dass er die Verankerungselemente ohne hinderliche Fremdteile anbringen kann, dass er ein dem Abstand der Verankerungselemente angepasstes Stabilisierungselement minimaler Baulänge auswählen und montieren kann und dass er die Verbindung zwischen Stabilisierungselement und Verankerungselement in einer ihm passenden Lage starr setzen kann.

Im folgenden wird die Erfindung anhand von Ausführungsbeispielen beschrieben. Es zeigen:

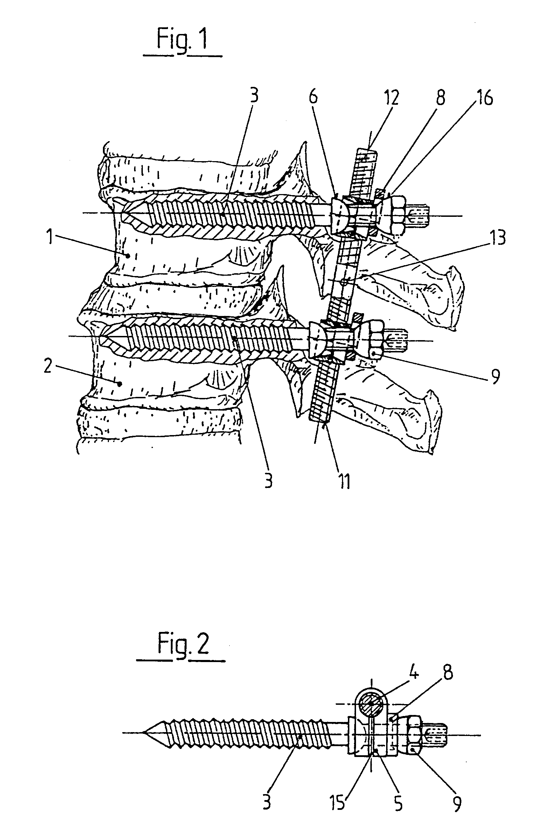

- Fig. 1: das Schnittbild einer Seitenansicht für die einseitige Fixierung zweier Wirbel mit Hilfe des neuen Implantates;
- Fig. 2: eine Draufsicht auf ein Implantat gemäss Fig. 1;
- Fig. 3: ein vergrössertes Schnittbild der Verbindung zwischen Verankerungselement und Stabilisierungselement gemäss Fig. 2 und
- Fig. 4: ein vergrössertes Schnittbild der Verbindung zwischen Verankerungselement und Stabilisierungselement in einer zu Fig. 3 abweichenden Ausführungsform.

Zur Fixierung zwischen zwei Wirbeln 1, 2 ist jeweils eine Knochenschraube 3 in ähnlicher Lage mittels eines Innensechskants 17 fest eingeschraubt und über ein Stabilisierungselement 4 mit der anderen Knochenschraube 3 starr verbunden. Das Stabilisierungselement 4 ist als Leitspindel mit Rechts- und Linksgewinde 12, 11 ausgeführt, die jeweils in der geschlitzten Gewindebohrung einer Klemmbride 5 läuft, derart, dass eine Klemmkraft über den Schlitz 15 die Spindel 4 festsetzt. Eine Klemmkraft wird erzeugt, indem über den Schlitz 15 und quer zu der Spindelachse ein Klemmdurchbruch 7 besteht, durch den der als Zuganker ausgebildete hintere Teil der Knochenschraube 3 ragt, der seinerseits ein Gewinde aufweist, auf dem eine Klemmutter 9 läuft. Durch Anziehen der Klemmutter 9 wird die Klemmbride 5 mit dem Aussenrand ihres Klemmdurchbruchs 7 auf einem kugelähnlich bombierten Kragen 6 der Knochenschraube 3 in ihrer momentanen Lage positioniert und über Reibungskräfte festgehalten, während die Spindel 4 in ihrer Drehbewegung blockiert wird. Das Verstellen der Leitspindel 4 geschieht mit einem formschlüssigen Werkzeug und an der Bohrung 13 wird die Spindel 4 mit Draht zusätzlich gegen Verdrehung gesichert.

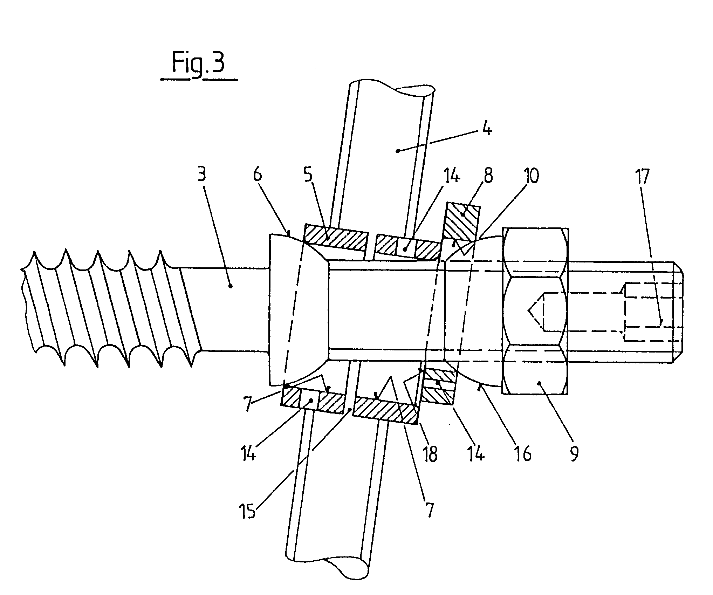

In Fig. 3 überträgt beim Anziehen der Klemmutter 9 eine Unterlagsscheibe 8 die Klemmkraft, indem sie mit ihrer Berührungsfläche 18 zur Bride 5 deren äussere Neigung annimmt und mit der Aussenkante von ihrem Durchbruch 10 der kugelähnlich bombierten Fläche 16 in der Ebene, die sich durch die Neigung ergibt, folgt, bis die Klemmung eintritt. Eine Selbsthemmung der Klemmung tritt dann ein, wenn mit dem Aufbringen der Klemmkraft so grosse Haftkräfte in der Berührungsfläche 18 zwischen Unterlagsscheibe 8 und Bride 5 auftreten, dass auch eine von aussen eingeleitete Verkleinerung der Neigung verhindert wird.

Es ergeben sich somit zwei überlagerte Verstellbereiche, einmal die bis zur Klemmung unabhängige Drehung von Bride 5 auf der Schraubenlinie der Spindel 4 und zum anderen die Schrägstellung des hinteren Teils der Knochenschraube im Durchbruch 7 der Bride 5. Theoretisch würde es genügen, die Unterlagsscheibe 8 in der sich ergebenden Schräglage mit der Bride 5 zu verstiften, um Verschiebungen in der gemeinsamen Berührungsfläche 18 zu verhindern und um Selbsthemmung der Verbindung in Schräglage sicherzustellen. Für den praktischen Einsatz und eine einfache Korrektur der Einstellung wird eine Erhöhung der Reibung in der Berührungsfläche 18 vorgezogen, wobei dies durch die Werkstoffwahl der Oberflächen und/oder durch Aufrauhen der Oberflächen und/oder durch eine ineinander greifende Textur der Oberflächen geschehen kann.

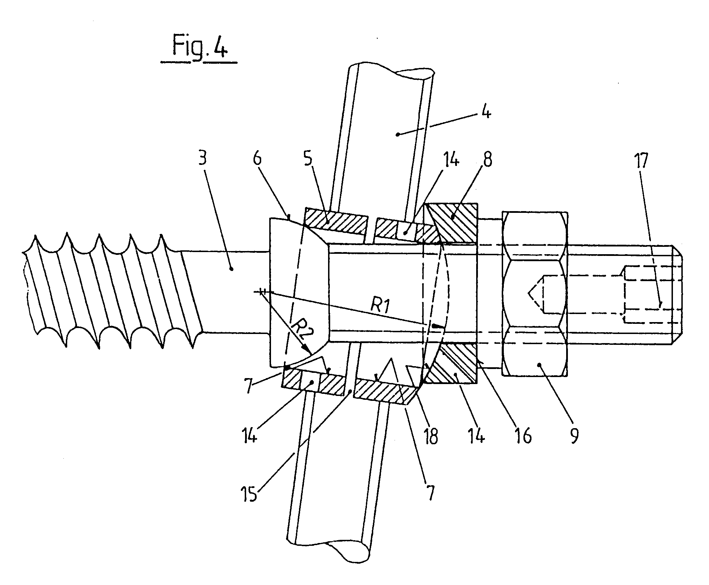

In Fig. 4 ist eine weitere Ausführungsform gezeigt, in der die Berührungsflächen 18, 16 abweichend zu Fig. 3 gestaltet sind. Die für die Unterlagsscheibe 8 und die Klemmbride 5 gemeinsame Berührungsfläche 18 für beide Elemente ist als Ausschnitt einer Kugelfläche mit Radius R1 ausgeführt, die bei Montage ohne Neigung einen annähernd gemeinsamen Mittelpunkt mit der als Kugelfläche R2 ausgeführten Fläche 6 aufweist. Die Berührungsfläche 16 der Klemmutter 9 zur Unterlagsscheibe 8 ist als axiale Anpressschulter ausgeführt, während die Unterlagsscheibe 8 zur Achse der Knochenschraube 3 zentriert ist. Eine Erhöhung der Reibung in der Berührungsfläche 18 ist hier ebenso sinnvoll, um den Anwendungsbereich für die zulässige Neigung mit Selbsthemmung der Klemmung zu vergrössern.

Für den Operateur wird es als vorteilhaft angesehen, wenn die Verbindung zwischen Leitspindel 4 und Knochenschraube 6 in jeder schwenkbaren Schräglage selbsthemmend starr ist. Dies wird erreicht, indem der schwenkbare Spielraum der Knochenschraube 3 im Klemmdurchbruch 7 so klein gehalten ist, dass die Reibung in der Berührungsfläche 18 selbsthemmend wirkt. Der Innensechskant 17 ist als Sackloch in der Knochenschraube 3 fortgesetzt, um mit einem Steckwerkzeug zusätzlich ein auf die Schräglage wirkendes Biegemoment auf die Knochenschraube 3 beim Anziehen der Klemmutter 9 auszuüben.

Klemmbride 5 und Unterlagsscheibe 8 weisen Verbindungsöffnungen 14 auf, die eine direktere Verbindung zwischen aussenliegenden und innenliegenden Flächen zur Verhinderung "toter" und am Stoffwechsel nicht beteiligter Räume ermöglichen.

## Patentansprüche

1. Implantat zur Fixierung zweier Wirbel (1, 2) relativ zueinander, bestehend aus zwei jeweils in die beiden Wirbel (1, 2) eingreifenden Verankerungselementen (3), die über ein den Abstand der Verankerungselemente (3) überbrückendes Stabilisierungselement (4) verbunden sind, wobei das Stabilisierungselement (4) innerhalb eines vorgebenen Bereiches in einem beliebigen Winkel zum Verankerungselement (3) fixierbar ist, dadurch gekennzeichnet, dass eine Klemmbride (5) einerseits mit einem Aussenrand ihres Klemmdurchbruchs (7) auf einer kugelähnlich bombierten Fläche (6) des Verankerungselementes (3) aufsitzt und über eine ebene mit einer Unterlagsscheibe (8) gemeinsame Berührungsfläche (18) mit dieser in Kontakt ist, wobei die Unterlagsscheibe (8) mit dem Rand ihres Durchbruchs (10) auf einer kugelähnlich bombierten Berührungsfläche (16) der Klemmutter (9) angepresst ist und andererseits die Klemmbride (5) das Stabilisierungselement (4) durch Klemmen festhält, indem die Klemmbride (5) quer zum Klemmdurchbruch (7) einen schlitzähnlichen Einschnitt (15) bis zur Durchdringung mit dem Stabilisierungselement (4) aufweist, der eine Deformation bis zur Klemmung des Stabilisierungselementes (4) unter der Einwirkung der Klemmmutter (9) zulässt.

2. Implantat zur Fixierung zweier Wirbel (1, 2) relativ zueinander, bestehend aus zwei jeweils in die beiden Wirbel (1, 2) eingreifenden Verankerungselementen (3), die über ein den Abstand der Verankerungselemente (3) überbrückendes Stabilisierungselement (4) verbunden sind, wobei das Stabilisierungselement (4) innerhalb eines vorgegebenen Bereiches in einem beliebigen Winkel zum Verankerungselement (3) fixierbar ist, dadurch gekennzeichnet, dass eine Klemmbride (5) einerseits mit einem Aussenrand ihres Klemmdurchbruchs (7) auf einer kugelähnlich bombierten Fläche (6) des Verankerungselementes (3) aufsitzt und über eine kugelförmig bombierte mit einer Unterlagsscheibe (8) gemeinsame Berührungsfläche (18) angepresst ist, welche ihrerseits von einer Klemmutter (9) angepresst ist, wobei die gemeinsame kugelförmig bombierte Berührungsfläche (18) sowie die kugelförmig bombierte Fläche (6) des Verankerungselementes (3) bei geklemmter Lage ihren Mittelpunkt annähernd am gleichen Ort aufweisen und andererseits die Klemmbride (5) das Stabilisierungselement (4) durch Klemmen festhält, indem die Klemmbride (5) quer zum Klemmdurchbruch (7) einen schlitzähnlichen Einschnitt (15) bis zur Durchdringung mit dem Stabilisierungselement (4) aufweist, der eine Deformation bis zur Klemmung des Stabilisierungselementes (4) unter der Einwirkung der Klemmutter (9) zulässt.

3. Implantat nach Anspruch 1, dadurch gekennzeichnet, dass die Oberflächen in der Berührungsfläche (18) zwischen Klemmbride (5) und Unterlagsscheibe (8) eine erhöhte Reibung durch Aufrauhen und/oder durch Werkstoffwahl und/oder durch eine ineinandergreifende Textur aufweisen.

4. Implantat nach einem der Ansprüche 1 oder 3, dadurch gekennzeichnet, dass die mit dem Klemmdurchbruch (7) maximal zugelassene Schräglage des Verankerungselementes (3) im Zusammenwirken mit der Reibung in der Berührungsfläche (18) so begrenzt ist, dass die Verbindung zwischen Stabilisierungselement (4) und dem Verankerungselement (3) mit Sicherheit selbsthemmend ist.

5. Implantat nach einem der Ansprüche 1 oder 2, dadurch gekennzeichnet, dass die Klemmbride (5) zu ihrem Klemmdurchbruch (7) und/oder die Unterlagsscheibe (8) zum Innenraum Verbindungsöffnungen (14) aufweist.

## Claims

1. An implant for fixing two vertebrae (1, 2) relatively to one another and comprising two fixing elements (3) which engage one each in the two vertebrae (1, 2) and which are interconnected by way of a stabilizing element (4) bridging the gap between the fixing elements (3), the stabilising element (4) being fixable at any angle relatively to the fixing element (3) in a predetermined range, characterised in that a clamp (5) has an outside edge of its aperture (7) disposed on a spherically curved surface (6) of the fixing element (3) and is in contact therewith by way of a flat contact surface (18) common to a washer (8), the edge of the aperture (10) of the washer (8) being pressed on a spherically curved contact surface (16) of the clamp nut (5), the clamp (5) also retaining the stabilising element (4) by clamping, the clamp (5) being formed transversely to its aperture (7) with a slot-like incision (15) extending to where the stabilising element (4) extends through, such incision permitting deformation until the stabilising element (4) is clamped by means of the clamping nut (9).

2. An implant for fixing two vertebrae (1, 2) relatively to one another and comprising two fixing elements (3) which engage one each in the two vertebrae (1, 2) and which are interconnected by way of a stabilising element (4) bridging the gap between the fixing elements (3), the stabilising element (4) being fixable at any angle relatively to the fixing element (3) in a predetermined range, characterised in that a clamp (5) has an outside edge of its aperture (7) disposed on a spherically curved surface (6) of the fixing element (3) and is subjected to pressure via a spherically curved contact surface (18) common to a washer (8), said contact surface (18) in turn being subjected to the pressure of a clamping nut (9), the common spherically curved contact surface (18) and the spherically curved surface (6) of the fixing element (3) having their centres approximately in the same place in the clamped position, while the clamp (5) retains the stabilising element (4) by clamping, the clamp (5) being formed transversely to the aperture (7) with a slot-like incision (15) extending to where the stabilising element (4) extends through, such incision permitting deformation until the stabilising element (4) is clamped by means of the clamping nut (9).

3. An implant according to claim 1, characterised in that the surfaces in the contact surface (18) between the clamp (5) and the washer (8) have elevated friction as a result of being roughened and/or as a result of the choice of material and/or as a result of a meshing texture.

4. An implant according to any of claims 1 - 3, characterised in that the maximum inclination of the fixing element (3) permitted by the clamp aperture (7) is so limited in co-operation with the friction in the contact surface (18) that the connection between the stabilising element (4) and the fixing element (3) is reliably self-locking.

5. An implant according to claim 1 or 2, characterised in that the clamp (5) is formed with connecting apertures (14) extending to its aperture (7) and/or the washer (8) is formed with connecting apertures (14) extending to the interior.

## Revendications

1. Implant pour la fixation de deux vertèbres (1, 2) l'une par rapport à l'autre, constitué de deux éléments d'ancrage (3) s'engageant chacun dans les deux vertèbres (1, 2), qui sont reliés par un élément de stabilisation (4) pontant la distance des éléments d'ancrage (3), l'élément de stabilisation (4) pouvant être fixé à l'intérieur d'une zone donnée, sous un angle quelconque par rapport à l'élément d'ancrage (3), caractérisé en ce qu'une bride de serrage (5) repose d'une part avec un bord extérieur de son ajour de serrage (7) sur une surface (6), bombée à la manière d'une bille, de l'élément d'ancrage (3) et est en contact, par une surface de contact (18) plane, commune à une rondelle (8), avec celle-ci, la rondelle (8) étant pressée avec le bord de son ajour (10) sur une surface de contact (16), bombée à la manière d'une bille, de l'écrou de serrage (9), et d'autre part la bride de serrage (5) fixant l'élément de stabilisation (4) par serrage, en ce que la bride de serrage (5) présente, transversalement à l'ajour de serrage (7), une entaille (15) semblable à une fente jusqu'à pénétration avec l'élément de stabilisation (4) qui autorise une déformation jusqu'au serrage de l'élément de stabilisation (4), sous l'effet de l'écrou de serrage (9).

2. Implant pour la fixation de deux vertèbres (1, 2) l'une par rapport à l'autre, constitué de deux éléments d'ancrage (3) s'engageant chacun dans les deux vertèbres (1, 2), qui sont reliés par un élément de stabilisation (4) pontant la distance des éléments d'ancrage (3), l'élément de stabilisation (4) pouvant être fixé à l'intérieur d'une zone donnée, sous un angle quelconque par rapport à l'élément d'ancrage (3), caractérisé en ce qu'une bride de serrage (5) repose d'une part avec un bord extérieur de son ajour de serrage (7) sur une surface (6), bombée à la manière d'une bille, de l'élément d'ancrage (3) et est pressée, par une surface de contact (18) bombée en forme de bille, commune à une rondelle (8), laquelle surface est de son côté pressée par un écrou de serrage (9), la surface de contact (18) bombée en forme de bille, commune, ainsi que la surface (6) bombée en forme de bille de l'élément d'ancrage (3) ayant, en position serrée, leur centre à peu près au même point et d'autre part la bride de serrage (5) fixant l'élément de stabilisation (4) par serrage, en ce que la bride de serrage (5) présente, transversalement à l'ajour de serrage (7), une entaille (15) semblable à une fente jusqu'à pénétration avec l'élément de stabilisation (4) qui autorise une déformation jusqu'au serrage de l'élément de stabilisation (4), sous l'effet de l'écrou de serrage (9).

3. Implant selon la revendication 1, caractérisé en ce que les surfaces dans la surface de contact (18), entre la bride de serrage (5) et la rondelle (8), présentent une friction accrue par rugosité et/ou par choix des matériaux et/ou par une texture d'imbricatioon.

4. Implant selon l'une des revendications 1 ou 3, caractérisé en ce que la position oblique de l'élément d'ancrage (3), autorisée au maximum avec l'ajour de serrage (7), en coopération avec la friction dans la surface de contact (18), est limitée de manière que la liaison entre l'élément de stabilisation (4) et l'élément d'ancrage (3) soit autobloquante en toute certitude.

5. Implant selon l'une des revendications 1 ou 2, caractérisé en ce que la bride de serrage (5) présente des ouvertures de liaison (14) vers son ajour de serrage (7) et/ou la rondelle (8) présente des ouvertures de liaison (14) vers l'intérieur.
